(19) Europäisches Patentamt
European Patent Office
Office européen des brevets

(11) **EP 3 675 738 B1**

(12) **EUROPEAN PATENT SPECIFICATION**

(45) Date of publication and mention
of the grant of the patent:
**20.11.2024 Bulletin 2024/47**

(21) Application number: **18766343.0**

(22) Date of filing: **28.08.2018**

(51) International Patent Classification (IPC):
**A61B 5/16** *(2006.01)*     **A61B 5/24** *(2021.01)*
**A61B 5/024** *(2006.01)*     **G16H 50/20** *(2018.01)*

(52) Cooperative Patent Classification (CPC):
**A61B 5/165; A61B 5/02405; A61B 5/316;**
**A61B 5/349; A61B 5/366; A61B 5/7264;**
**G16H 50/20; G16H 50/30; G16H 50/70**

(86) International application number:
**PCT/IB2018/056558**

(87) International publication number:
**WO 2019/043579 (07.03.2019 Gazette 2019/10)**

(54) **METHOD AND DEVICE FOR DETECTING STRESS USING BEAT-TO-BEAT ECG FEATURES**

VERFAHREN UND VORRICHTUNG ZUR DETEKTION VON SPANNUNGEN UNTER
VERWENDUNG VON SCHLAG-ZU-SCHLAG-EKG-FUNKTIONEN

PROCÉDÉ ET DISPOSITIF DE DÉTECTION DE STRESS À L'AIDE DE CARACTÉRISTIQUES D'ECG
BATTEMENT À BATTEMENT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **28.08.2017 EP 17188217**
**22.02.2018 PT 2018110584**
**23.02.2018 EP 18158483**

(43) Date of publication of application:
**08.07.2020 Bulletin 2020/28**

(73) Proprietor: **INESC TEC - Instituto de Engenharia de**
**Sistemas e Computadores, Tecnologia e Ciência**
**4200-465 Porto (PT)**

(72) Inventors:
• **TRIGUEIROS DA SILVA CUNHA, João Paulo**
**4200-465 Porto (PT)**
• **SANTOS PAIVA, Joana Isabel**
**4520-022 Escapães (PT)**

(74) Representative: **Patentree**
**Edificio Net**
**Rua de Salazares, 842**
**4149-002 Porto (PT)**

(56) References cited:
**EP-A1- 3 449 828     US-A1- 2015 257 668**

• **AXMAN DUSTIN ET AL: "Beat-to-beat ECG**
**features for time resolution improvements in**
**stress detection", 2017 25TH EUROPEAN**
**SIGNAL PROCESSING CONFERENCE**
**(EUSIPCO), EURASIP, 28 August 2017**
**(2017-08-28), pages 1290 - 1294, XP033236145,**
**[retrieved on 20171023], DOI:**
**10.23919/EUSIPCO.2017.8081416**
• **PAIVA JOANA S ET AL: "Changes in ST, QT and**
**RR ECG intervals during acute stress in**
**firefighters: A pilot study", 2016 38TH ANNUAL**
**INTERNATIONAL CONFERENCE OF THE IEEE**
**ENGINEERING IN MEDICINE AND BIOLOGY**
**SOCIETY (EMBC), IEEE, 16 August 2016**
**(2016-08-16), pages 3378 - 3381, XP032979897,**
**DOI: 10.1109/EMBC.2016.7591452**
• **MARCO LAURINO ET AL: "Comparative study of**
**morphological ECG features classificators: An**
**application on athletes undergone to acute**
**physical stress", INTELLIGENT SYSTEMS**
**DESIGN AND APPLICATIONS (ISDA), 2011 11TH**
**INTERNATIONAL CONFERENCE ON, IEEE, 22**
**November 2011 (2011-11-22), pages 242 - 246,**
**XP032086061, ISBN: 978-1-4577-1676-8, DOI:**
**10.1109/ISDA.2011.6121662**

• KESHAN N ET AL: "Machine learning for stress detection from ECG signals in automobile drivers", 2015 IEEE INTERNATIONAL CONFERENCE ON BIG DATA (BIG DATA), IEEE, 29 October 2015 (2015-10-29), pages 2661 - 2669, XP032837278, DOI: 10.1109/BIGDATA.2015.7364066

## Description

### Technical field

The present disclosure relates to a device for detecting stress and a method to calculate an indicator indicative of stress using beat-to-beat ECG features.

### Background

Recent years have seen a surge in the popularity and convenience of devices that collect physiological data. This has led to numerous efforts to use this data for a wide breadth of pertinent classification tasks such as the detection of cardiac arrhythmia, stress, sleep stages, drug use, and emotion [2]. Success in these classification tasks would have enormously broad and beneficial applications in many areas of public health including: preventing car accidents, increasing worker efficiency, mitigating health problems, monitoring drug use more effectively and improving Human-Computer Interaction [2].

Firefighting is one of the careers upon which stress has the largest negative impact. Firefighters are consistently exposed to stressful and fatiguing situations, giving them a higher risk of coronary diseases which account for a large percentage of deaths among these professionals.

Air Traffic Control (ATC) is also a very complex process [20]. It depends to a large degree on human abilities and it encompasses high levels of responsibility not only with regard to risking lives but also for the high economical costs of aeronautical activities. Much of the Air Traffic Controllers (ATCs) work complexity is characterized by unplanned tasks that require constantly re-plans in response to weather, traffic management initiatives, airport construction, maintenance activities, and other events [20].

The above reasons make both FFs and ATCs prime candidates for stress sensing experiments. In this way, simultaneously analyzing subject's perceived stress levels and physiological signals such as electrocardiogram (ECG) in both populations and other first responders, is the first step towards a general stress sensing solution, applicable to all contexts [2,4]. Since acute stress events induce physiological responses by our cardiovascular and neuroendocrine systems, ECG-derived features both in time and frequency domains have been widely used for stress monitoring and are highly correlated with subject's stress and arousal state changes [4]. Indeed, numerous authors have been exploring the use of ECG features in a human affect context. Most of these groups focus primarily on affect detection using Window-derived Heart Rate Variability (W-HRV) features [7]. However, this latter method has drawbacks. While the use of a window allows for a wide range of features to be used, including spectral features, these windows are usually 80 to 300 seconds which deteriorates the temporal resolution and increases the computational complexity of the detector in which such windows are used.

Methods for detecting stress should be evaluated not only accuracy, but also time resolution and computational rapidity of each method. Such metrics could be of high importance, since stressful events have been linked to abnormalities in cardiovascular functions (e.g. arrhythmias, cardiomyopathy, etc) in healthy and non-healthy persons, making prompt stress detection very desirable. Such methods are known from PAIVA JOANA S ET AL, "Changes in ST, QT and RR ECG intervals during acute stress in firefighters: A pilot study", (2016), 38TH ANNUAL INTERNATIONAL CONFERENCE OF THE IEEE ENGINEERING IN MEDICINE AND BIOLOGY SOCIETY (EMBC), IEEE, 16 August 2016 (2016-08-16), pages 3378-3381, DOI: 10.1109/EMBC.2016.7591452.

These facts are disclosed in order to illustrate the technical problem addressed by the present disclosure.

### General Description

The invention is defined in the appended claims. In stress sensing, Window-derived Heart Rate Variability (W-HRV) methods are by far the most heavily used feature extraction methods. However, these W-HRV methods come with a variety of tradeoffs that motivate the development of alternative methods in stress sensing. We compare the presently disclosed method of using heart heat morphology (HBM) features for stress sensing to the traditional W-HRV method for feature extraction. In order to adequately evaluate these methods we conducted a Trier Social Stress Test (TSST) to elicit stress in a group of 13 firefighters and 11 Air Traffic Controllers (ATCs) while recording their ECG, actigraphy, and psychological self-assessment measures. We utilize the data from this experiment to analyze both feature extraction methods in terms of computational complexity, detection resolution performance, and event localization performance for each type of population separately (FFs and ATCs). Our results show that each method has an ideal niche for its use in stress sensing. W-HRV shows higher performance in stress event identification (7% higher in accuracy, 17% in precision and 22% in F1 score) but needs much more time to detect its presence than HBM (95% more - 16sec vs. 0.79sec). Thus the presently disclosed method, HBM, tends to be more effective in an online "fast" stress detection context where W-HRV shows to be more suitable for offline post processing scenarios. These results were reproducible on both occupational groups. Furthermore, the presently disclosed method, HBM, requires much less computational power than W-HRV.

The disclosure includes a method for calculating an indicator indicative of stress.

It is disclosed a non-transitory storage media including computer program instructions which, when executed by a processor, cause the processor to carry out a method for on-line determining an indicator indicative of stress using individual heart beat ECG features of data acquired from an individual subject, according to claim 1.

According to the invention, the fiducial heart beat features further comprise ST, QR, STc and RR.

According to the invention, the fiducial heart beat features further comprise QRS and STinterval, in particular further comprising QTc.

It is also disclosed a method for on-line determining of an indicator of stress using individual heart beat ECG features of data acquired from a subject, comprising:

obtaining a data sample of each heart beat individually from the acquired data;
calculating the fiducial features RR, QR, RT, STinterval, STc, QTinterval, QTc, ST and QRS from each said data sample;
classifying each data sample as indicative of stressed or not-stressed, using a pretrained classifier which was previously trained using the fiducial heart beat features RR, QR, RT, STinterval, STc, QTinterval, QTc, ST and QRS from previously acquired reference data samples from individual heart beats,
determining the indicator of stress as detected when at least one data sample is classified as indicative of stressed.

According to the invention, the used fiducial features consist of the fiducial features: RR, QR, RT, STc, QTinterval, ST, QRS and STinterval.

In an embodiment, the indication of stress is determined as detected when one data sample is classified as indicative of stressed over a time duration of only one heart beat and the RR distance between said one heart beat and the previous heart beat.

In an embodiment, the indication of stress is determined as detected when 1-50, 1-20, 1-10, 1-5, 1-2, or just 1 data samples are classified as indicative of stressed.

In an embodiment, the indication of stress is determined as detected when a majority of data samples are classified as indicative of stressed over a predetermined duration of the acquired data.

In an embodiment, the indication of stress is determined as detected when a predetermined number of consecutive heart beats are classified as stressed, in particular 50, 20, 10, 5 or 2 consecutive heart beats are classified as stressed.

According to the invention, the pretrained classifier is a Linear Support Vector Machine (SVM), a Kernel Support Vector Machine (K-SVM), a K-Nearest Neighbor (K-NN), or a Random Forest classifier.

In an embodiment, the pretrained classifier is a Random Forest Classifier.

In an embodiment, the determining of the indication of stress comprises detecting the indication of stress under a predetermined time interval after a heart beat classified as indicative of stress.

The disclosure includes calculating said indicator indicative of stress for operational purposes, in particular for fire-fighting and air traffic controlling operational purposes.

It is also disclosed a device for on-line detecting stress using individual heart beat ECG features of data acquired from an individual subject, comprising a data processor and data storage media, according to claim 6.

In an embodiment, the detecting of stress comprises detecting stress under a predetermined time interval between a heart beat classified as stressed and the beginning of a stress interval of said subject.

In an embodiment, the device is a wearable device.

## Brief Description of the Drawings

The following figures provide preferred embodiments for illustrating the description.

Figure 1: Schematic representation of a diagram of an embodiment of the method. VAS - Visual Analogue Scales. TSST - Trier Social Stress Test, wherein 1 represents a start/end event, 2 represents an intermediate event, 3 represents visual scale, 4 represents self-reports.

Figure 2: Schematic representation of a portion of ECG from FF 2, with fiducial points Q, R, S and T; and points that contributed for extracting QT and ST intervals marked.

Figure 3: Schematic representation of a sketch of a heartbeat waveform illustrating six of the nine temporal intervals used in the classification task: QR segment; RT segment; ST interval; QT interval; ST segment and QRS segment.

**Figure 4:** Schematic representation of a summary of an embodiment of the classification method.

**Figure 5:** Schematic representation of a summary of an embodiment of the classification method used as a detection protocol.

## Detailed Description

The following pertains to methods of the disclosure, in particular to a description of a sample population dataset used in the present disclosure.

A dataset obtained from a population sample of 13 FFs and 11 ATCs with a high age variability (FFs: 3 female; age: 31 $\pm$ 11 years; ATCs: 3 female; age: 47 $\pm$ 6 years) was used in a study for demonstrating the advantages of the present disclosure. Data records from subjects with a history of cardiovascular disease and/or prescription cardiovascular-related drug use were not included.

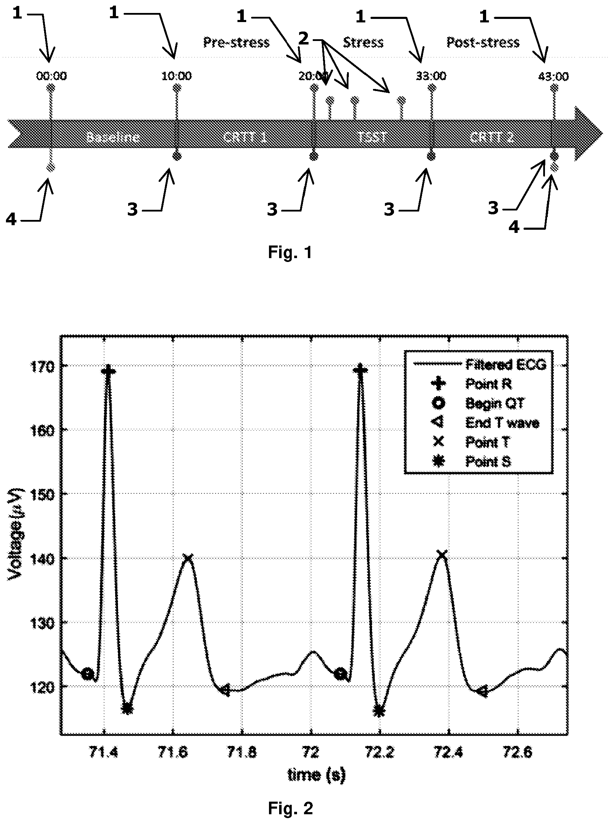

The following pertains to a description of the laboratory protocol of this study. The applied laboratory protocol (figure 1) was conducted in a previous study by our laboratory and proved to be a suitable protocol to induce acute stress in FFs [4]. ECG signals were continuously acquired throughout the duration of the experiment (approx. 1 hour) using the VitalJacket® [9] (VJ) at 500 Hz from a single lead. The VJ is a wearable bio-monitoring platform (in form of a t-shirt) able to collect ECG signals in a real-time manner, without affecting daily activities of users. It also contains a 3-axis Accelerometer system, allowing ECG signals correction for actigraphy profiles.

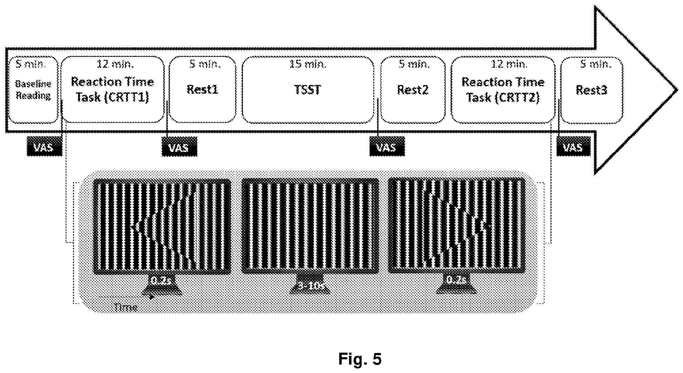

The laboratory protocol (see Fig. 5) performed by volunteers was composed of 3 main tasks during which they were comfortably sat in a chair. For evaluating the impact of stress in cognitive performance and after collecting an ECG baseline reading, a 2-choice reaction time task (CRTT1) [10] was conducted. This task required participants to respond to a stimulus, by clicking in a button, as fast as possible. The stimuli consisted in three different types of targets: (left screen) an arrowhead pointing to the left on top of a square both filled with a sinewave grating pattern; (right screen) the same stimulus, but with the arrowhead directed to the right; (middle screen) the grating square only (without an arrowhead) which remained in the center of the screen, between the appearance of each stimulus along the task - as illustrated in Figure 1. Following this, the Trier Social Stress Test (TSST) [8], a gold-standard psychological stress assessment procedure, was applied. After subjects were exposed to the stress condition, they performed again the simple CRTT (CRTT2) described above. Visual Analog Scales (VAS) [11] were used for stress psychological self assessment after each main task (after CRTT1, after TSST and after CRTT2). Participants were allowed to rest between each main event. All the ECG-based samples not belonging to the "TSST" event were labeled as "non-stress" and remaining ones as "stress".

The laboratory protocol performed by volunteers was composed of 3 main tasks during which they were comfortably sat in a chair. For evaluating the impact of stress in cognitive performance, a 2-choice reaction time task (CRTT) [10], was conducted. Following this, the Trier Social Stress Test (TSST) [8], a gold-standard psychological stress assessment procedure, was applied. After subjects were exposed to the stress condition, they performed again the simple CRTT (CRTT2) described above. Visual Analog Scales (VAS) were used for stress psychological self assessment after each main task (after CRTT1, after TSST and after CRTT2).

The following pertains to the ECG processing and features extraction. Since the primary method for feature extraction in the literature uses a W-HRV approach [7], where features are extracted from a fixed-length time interval with each sample representing a different shift of this interval, we compared the accuracy achieved in the proposed classification problem using W-HRV versus the disclosed HBM method both in FFs and ATCs. In HBM, each heartbeat waveform is treated as a separate sample.

In order to compare the two methods, we created a separate set of labelled samples for each method - see table I, for the presently used features in contrast with those used in prior art documents.

Table I: Enumeration of the features used in the classification task for each type.

| HeartBeat Morphology (HBM) Features [4], [12] | | Windowed Heart Rate Variability (W-HRV) Features [6], [7] |
|---|---|---|
| 1. $RR$ segment<br>2. $QR$ segment<br>3. $RT$ segment<br>4. $ST$ interval<br>5. $ST_C$ interval<br>6. $QT$ interval | Frequency Domain | 1. Spectral power in [0-0.015] Hz<br>2. Spectral power in [0.015-0.025] Hz band<br>3. Spectral power in [0.025-0.050] Hz band<br>4. Spectral power in [0.050-0.120] Hz band<br>5. Spectral power in [0.120-0.300] Hz band<br>6. Spectral power in [0.300-0.400] Hz band |
| 7. $QT_C$ interval<br>8. $ST$ segment<br>9. $QRS$ segment | Time Domain | 7. AVNN (average of NN-intervals)<br>8. SDNN (standard deviation of $NN$-intervals)<br>9. rMSSD (square root of the mean squared difference of successive $NN$ intervals)<br>10. pNN50 (number of pairs of successive $NN$ intervals that differ by more than 50 ms)<br>11. RMS of the mean of the square of $NN$ intervals |

The following pertains to presently disclosed method, HBM, features extraction. ECG heartbeats acquired during the different stages of the protocol were considered as samples with the temporal metrics extracted from each of these heartbeats as the features that characterize each respective sample of the dataset. ECG heartbeats (dataset samples) were therefore labelled as belonging to a "stressful" or "non-stressful" event according to the protocol stage in which they were acquired. Only the heartbeats that were collected in the TSST portion of the experiment were labelled as in the positive class, as per [4], with all others labelled as being in the negative class.

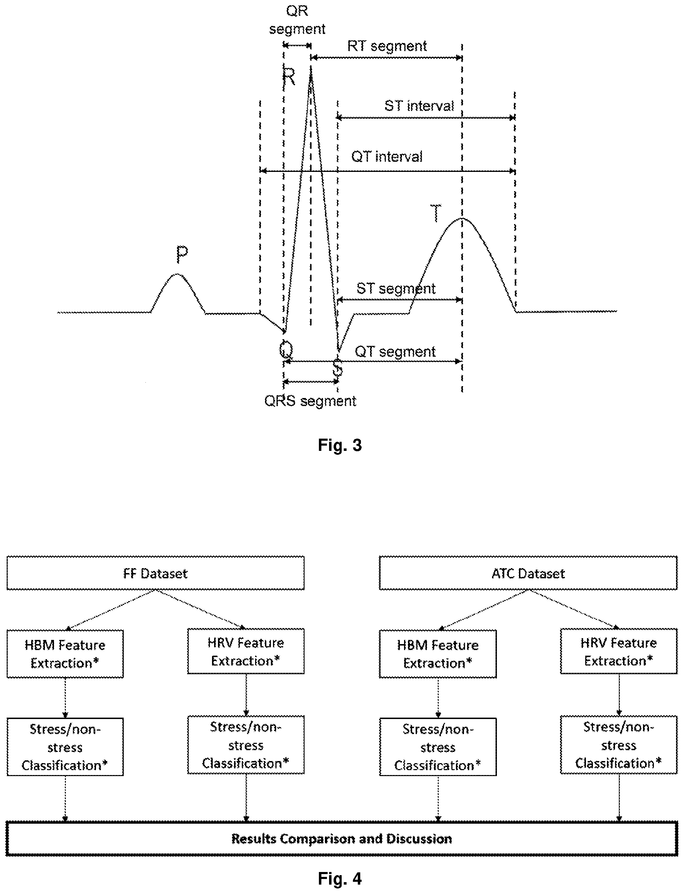

A set of nine features was extracted from each heartbeat waveform. The features used in this approach were based on temporal distances between fiducial points Q, R, S and T and were extracted using a ECG morphology-based patent pending [12, 21] processing scheme adopted in our previous study [4] - see figures 2 and 3. R points were the first fiducials to be located, using the widely known Pan Tompkins algorithm [13]. Considering that existing literature shows that the best method for detecting ECG fiducial points is based on low order polynomial filtering, the remaining fiducials - Q, S and T - were located after applying a second order Butterworth lowpass filter with a cut off frequency of 10 Hz to the raw signal. Fiducial points were discovered based on previously established physiological time intervals [15]. The Q points were identified by computing the signal derivative considering a time window of 0.10 seconds before each R point. The last peak within this time window was marked as point Q for each heartbeat. Point S was located by applying a similar method, also based on signal derivatives. The first temporal mark, at which the derivative changed from negative to positive values, 0.05 seconds after the R point, was assigned as the point S. For locating the peak of the T wave, it was determined the last temporal index where the derivative of the signal changed from positive to negative values, within a time window of 0.05 to 0.40 seconds after each QRS complex, for each heartbeat.

QR, RT, ST and QRS segments were calculated as depicted in figure 3. RR intervals were defined as the interval between two consecutive R points. Contrary to the prior art, R is used as a temporal feature and in a less complex way, also less computationally heavy. The index of the beginning of QT was computed as the last point where the derivative changed from positive to negative in a time window of 0.03 seconds before each Q point. The end of the T wave was computed as the index corresponding to the last point at which the signal derivative changed from negative to positive values within 0.15 seconds after the T peak. $ST_C$ and $QT_C$ intervals were also included. These are the ST and QT intervals corrected for the interference of heart rate for each heartbeat, using the *Bazett Formula* [16]:

$$QT_C = \frac{QT}{\sqrt{RR}} \; , ST_C = \frac{ST}{\sqrt{RR}}$$

Through this process the QR, RT, ST, RR and QRS segments, as well as the ST, QT, $ST_C$ and $QT_C$ intervals were calculated for each heartbeat. The QT segment was also initially considered but revealed to be highly correlated to several other features, it was excluded in the classification task, leaving the remaining 9 features used in the HBM method - please see table I.

Noisy HBs were removed after computing all the nine temporal distance measures, by identifying the HBs which did not satisfy the following conditions [15]:

$$QR \leq 0.075 \, s \; and \; 0.200 < QT_C < 0.360 \, s$$

The following pertains to W-HRV features extraction. A fixed window of length 80 seconds was chosen through 10-fold cross validation over our training set with a generic Random Forest classifier. Each window was labeled as belonging to a stress event if the majority of the heartbeat waveforms contained in this window were labeled as belonging to a stress event.

In accordance with the literature [7], the Lomb-Scargle Periodogram was calculated on the RR-intervals determined with Pan Tompkins algorithm [13] and 6 spectral features were extracted based on the power in each of several bands, described in Table I [7]. In addition to spectral features, we also extracted 5 time-based HRV features (described in Table I) [7].

A total of 11 W-HRV features were therefore extracted from each window, contrasting with the 9 features in the HBM method (Table I).

The following pertains to the classification task. We trained and tested on the same person data in our protocol to evaluate the effectiveness of each method with regard to within subject (rather than between subject) event sensing - please see figure 4. This was done by dividing the samples in each subject into 5 equally sized, random groups and using a leave-one-out testing scheme. This was done 5 times such that in the end, every sample in each subject's time series had a score associated with it. These scores were then un-permuted so as to rearrange them back into the temporally sequential order in which they had been collected. This gave us a vector of scores for every sample in each subject's time series, in order. Using this score vector and the ground truth vector, we compared HBM to W-HRV in 5 different standard metrics: Accuracy, Precision, Recall, F1 Score, and the Area Under the Receiver Operating Characteristic curve (AUROC).

Several classifiers were compared for use in evaluating the effectiveness of HBM features versus W-HRV features. Among these models were: Linear Support Vector Machines (SVM), Kernel Support Vector Machines (K-SVM), K-NN (K-Nearest Neighbor) and Random Forest.

We used 5-fold cross validation grid search to find the best parameters for each subject for each model. Number of models in Random Forest was chosen by grid search from 2 to 70 in increments of 2. SVM C parameter grid search was from $10^{-4}$ to $10^4$ over factors of 10. The K-SVM sigma grid search was from $10^{-4}$ to $10^4$ over factors of 10. K-NN K grid search was from 1 to 20 by increments of 2.

After this was done, the model with the highest average cross validation fold F1-Score for each subject was used for the remainder of our experimentation and evaluation for that respective subject. In every case, the model with the highest performance on the validation set was a Random Forest Classifier, differing in the number of trees used depending on the subject and the method. The number of predictors sampled from on each tree split was the square root of the number of total predictors.

We compared HBM with W-HRV in three main areas: computational complexity, stress localization, and stress detection, which are discussed below.

The HBM features require only a single pass through the ECG signal to detect fiducial points and perform the elementary operations necessary to derive the associated features making the entire HBM method $O(n)$ in computation.

For each new shift of the W-HRV, 16 seconds are removed from the end of the old window and the next 16 seconds of the time series are added onto the front of the new window. The Lomb Periodogram is derived for the entire new window. The Lomb Periodogram is O(nlog(n)). The remaining HRV features are O(n) making the entire W-HRV method O(nlog(n)+n) (O(nlog(n))).

This difference in computational complexity suggests different niches in stress event sensing where HBM features or W-HRV features shine. W-HRV features may not be as applicable to online sensing or wearable technology given the need for streamlined computation in these areas. Instead, W-HRV may be more suited for offline analysis of stress events.

Stress Localization is the process of determining the exact temporal bounds of a stress event. The nature of stress localization inherently gives equal importance to all samples within the stress event. With this in mind, in order to evaluate each method in this area, we used the metrics derived above. Average performance measures over all subjects (ATCs and FFs separately), for each method are shown in Table II.

Table II: Average test scores for each method and occupation.

|  |  | Accuracy | Precision | Recall | F1 Score | AUROC |
|---|---|---|---|---|---|---|
| FF | HBM | 0,837 | 0,703 | 0,595 | 0,628 | 0,845 |
|  | HRV | 0,928 | 0,865 | 0,800 | 0,814 | 0,958 |
|  | *difference* | *0,091* | *0,162* | *0,205* | *0,186* | *0,114* |
| ATC | HBM | 0,861 | 0,711 | 0,527 | 0,590 | 0,833 |
|  | HRV | 0,906 | 0,889 | 0,817 | 0,845 | 0,956 |
|  | *difference* | *0,045* | *0,178* | *0,289* | *0,255* | *0,123* |

HRV performance is better than HBM: difference of approx. 7% in accuracy, 17% in precision and 22% in F1 score (see table 2). The W-HRV method appears to be more effective for post processing the data offline when the goal is to determine the exact beginning, end, and duration of the stress event.

Unlike, stress localization, stress detection does not aim to determine exact bounds on the support of the event. Instead, it attempts to determine (as soon as possible) when the event begins, in an online fashion.

Let us define the Normalized Distance to Boundary (D), in percentage, as:

$$D = \frac{d}{L} \cdot 100$$

where d is the shortest distance between the predicted stress label and the beginning of the stress interval, and L is the length of the stress interval. In the following table, performance in terms of this measure is showed for each method and population in specific.

Table III: Normalized Distance to Boundary and Time Resolution.

|  |  | D (%) | Window Length (sec) | Time Resolution (sec) |
|---|---|---|---|---|
| **FF** | **HBM** | 1,55 | 0,79 * | 0,79 * |
|  | **HRV** | 2,03 | 80,00 | 16,00 ** |
|  | *difference* | 0,48 | 79,21 (99%) | 15,21 (95%) |
| **ATC** | **HBM** | 5,32 | 0.76 * | 0.76 * |
|  | **HRV** | 3,88 | 80,00 | 16,00 ** |
|  | *difference* | -1,44 | 79,24 (99%) | 15,24 (95%) |

\* average length of heart beat
\*\* window overlap of 80%

The following can be ascertained then:

- HRV and HBM have similar performance in detecting stress boundary (distance to stress boundary, metric D in table III).
- Needed window length to detect stress boundary is 98% smaller for HBM (see table III).
- Stress detection time resolution is 90% smaller for HBM (see table III).
- HRV is more accurate and precise but needs large windows and has low time resolution whereas HBM is less accurate and precise, but detects stress with a much larger time resolution, needing a very small window - only one heartbeat and its RR distance to the previous R peak, on average.
- If we are looking for a fast, cause/effect stress detector, HBM has much better characteristics than HRV. Nevertheless, if we want a precise and accurate stress detector and can sacrifice time resolution, HRV is a better method than HBM.

It is evident from this disclosure that W-HRV and HBM have specific applications in stress event sensing. W-HRV methods have slightly higher "accuracy" (F1 Score), yet they require more computation and do not achieve very good detection results.

In comparison, HBM methods require far less computation ((O(n)) computational complexity) and show excellent results in the area of detection. This makes the HBM method an excellent candidate for use in online processing and detection, while W-HRV methods are possibly more suitable for offline post-processing of the time series data. Therefore, each application should weigh the benefits of accurate detection with early detection. To provide a fair comparison, we have used a roughly equivalent number of features for both.

The features used in the detection method are found to be relevant for the intended result of detecting stress in a very short period of time, in different amounts. The weights in Table IV give an indication of this. Note that the features with the highest weights will not necessarily produce the best detectors, as that relationship is not linear or even straightforwardly predictable from these weights.

Table IV: Performance versus fiducial features.

| Features | Abs(weight) |
|---|---|
| RT | 0,713 |

(continued)

| Features | Abs(weight) |
|---|---|
| RR | 0,456 |
| QT interval | 0,286 |
| ST | 0,266 |
| QR | 0,229 |
| STc | 0,220 |
| QS | 0,125 |
| ST interval | 0,113 |
| QTc | 0,047 |

The features used in the detection method are also relevant for the intended result of detecting stress in a very short period of time, ideally in one heartbeat period in a very few heartbeat periods. The table V illustrates different results obtained for different numbers and combinations of heartbeat fiducial features.

Table V: Performance versus fiducial features.

| Features | Mean Accuracy | SD Accuracy | Mean F1 | SD F1 |
|---|---|---|---|---|
| RT | 0,800 | 0,106 | 0,563 | 0,254 |
| RT + QT interval | 0,888 | 0,078 | 0,705 | 0,221 |
| RR + STc + QTc | 0.912 | 0,034 | 0.783 | 0,142 |
| RT + RR + QT interval | 0,892 | 0,069 | 0,725 | 0,190 |
| RT + RR + QT interval + ST + QR +STc | 0,934 | 0,031 | 0,809 | 0,179 |
| RT + RR + QT interval + ST + QR +STc + QS + ST interval | 0,936 | 0,027 | 0,826 | 0,130 |
| RT + RR + QT interval + ST + QR +STc + QS + ST interval + QTc | 0,940 | 0,028 | 0,826 | 0,130 |

It can be seen as how, for single heartbeat period detections, the use of a single feature provides rather inferior results when compared to the other possibilities. Using 2 features, like RT, QT interval, or 3 features like RR, STc, QTc, or like RT, RR, QT interval provides results that are acceptable in some use cases. Also, adding more features enabled higher quality results, providing improved accuracy for 6 features RT, RR, QT interval, ST, QR, STc, or even 8 features RT, RR, QT interval, ST, QR, STc, QS, ST interval.

Results using RT have provided uniformly better results. This was surprising as RR would have been a more promising initial candidate. Furthermore, QT interval also provided uniformly better results when combined with RT. In particular, the combination of RT, RR, QT interval, ST, QR, STc provided particularly good detection results for single heartbeat periods. Adding, subsequently, the features QS or QTc does not substantially improve the results.

The following references are of interest:

[2] J. Cunha, "PHealth and wearable technologies: A permanent challenge," Stud. Health Technol. Inform, vol. 177, pp. 185-195, 2012.

[4] J. Paiva, S. Rodrigues, and J. Cunha, "Changes in ST, QT and RR ECG intervals during acute stress in firefighters: A pilot study," in Engineering in Medicine and Biology Society (EMBC), 2016 IEEE 38th Annual International Conference of the. IEEE, 2016, pp. 3378-3381.

[7] A. Voss, R. Schroeder, A. Heitmann, A. Peters, and S. Perz, "Shortterm heart rate variability - influence of gender and age in healthy subjects," PloS one, vol. 10, no. 3, p. e0118308, 2015.

[8] M. Birkett, "The Trier Social Stress Test protocol for inducing psychological stress," Journal of visualized experiments: JoVE, vol. 19, no. 56, 2011.

[9] J. Cunha, B. Cunha, A. Pereira et al., "Vital-jacket: A wearable wireless vital signs monitor for patients' mobility in cardiology and sports," in Pervasive Computing Technologies for Healthcare, 2010 4th International Conference on. IEEE, 2010, pp. 1-2.

[10] J. Paiva, "Predicting lapses in attention: a study of brain oscillations, neural synchrony and eye measures," MSc Thesis, University of Coimbra, pp. 33-36, 2014.

[12] J. Cunha and J. Paiva, "Biometric Method and Device for Identifying a Person Through an Electrocardiogram (ECG) Waveform - ref. PT109357," 2016, PT109357.

[13] J. Pan and W. Tompkins, "A real-time QRS detection algorithm," Biomedical Engineering, IEEE Transactions on, vol. 32, no. 3, pp. 230-236, 1985.

[15] D. Clifford, "ECG statistics, noise, artifacts, and missing data," Advanced Methods and Tools for ECG Data Analysis, vol. 6, pp. 55-99, 2006.

[16] H. Bazett, "An analysis of the time-relations of electrocardiograms," Heart, vol. 7, pp. 353-370, 1920.

[20] Koros A., D.R.P.S., Panjwani G., Ingurgio V., D'Arcy J.F. Complexity in air traffic control towers: A field study part 1. Complexity factors; Virginia 2003; pp 1-125.

[21] Paiva JS, Dias D, Cunha JPS (2017) Beat-ID: Towards a computationally low-cost single heartbeat biometric identity check system based on electrocardiogram wave morphology. PLOS ONE 12(7): e0180942. https://doi.org/10.1371/journal.pone.0180942.

The term "comprising" whenever used in this document is intended to indicate the presence of stated features, integers, steps, components, but not to preclude the presence or addition of one or more other features, integers, steps, components or groups thereof.

Flow diagrams of particular embodiments of the presently disclosed methods are depicted in figures. The flow diagrams do not depict any particular means, rather the flow diagrams illustrate the functional information one of ordinary skill in the art requires to perform said methods required in accordance with the present disclosure.

It will be appreciated by those of ordinary skill in the art that unless otherwise indicated herein, the particular sequence of steps described is illustrative only and can be varied without departing from the disclosure. Thus, unless otherwise stated the steps described are so unordered meaning that, when possible, the steps can be performed in any convenient or desirable order.

Furthermore, it is to be understood that the invention is defined in the appended claims.

It is to be appreciated that certain embodiments of the disclosure as described herein may be incorporated as code (e.g., a software algorithm or program) residing in firmware and/or on computer useable medium having control logic for enabling execution on a computer system having a computer processor, such as any of the electronic data processors described herein. Such a computer system typically includes memory storage configured to provide output from execution of the code which configures a processor in accordance with the execution. The code can be arranged as firmware or software, and can be organized as a set of modules, including the various modules and algorithms described herein, such as discrete code modules, function calls, procedure calls or objects in an object-oriented programming environment. If implemented using modules, the code can comprise a single module or a plurality of modules that operate in cooperation with one another to configure the machine in which it is executed to perform the associated functions, as described herein.

The disclosure should not be seen in any way restricted to the embodiments described and a person with ordinary skill in the art will foresee many possibilities to modifications thereof. The above described embodiments are combinable. The following claims define the invention.

**Claims**

1. Non-transitory storage media including computer program instructions which, when executed by a processor, cause the processor to carry out a method for on-line determining an indicator indicative of stress using individual heart beat ECG features of data acquired from an individual subject, comprising:

    obtaining a data sample of each heart beat individually from the acquired data; calculating a set of fiducial heart beat features from each said data sample of an individual heart beat; classifying each data sample as indicative of stressed or not-stressed, using a pretrained classifier which was previously trained using the same fiducial heart beat features from previously acquired reference data samples each from individual heart beats of said individual subject; and
    determining the indicator of stress as detected when at least one data sample of an individual heart beat is classified as indicative of stressed,
    wherein the fiducial heart beat features comprise RT, ST, QR, STc, RR, QRS, QTc, STinterval and QTinterval, and
    wherein the pretrained classifier is a Linear Support Vector Machine (SVM), a Kernel Support Vector Machine (K-SVM), a K-Nearest Neighbor (K-NN), or a Random Forest classifier.

2. Non-transitory storage media according to the previous claim, wherein the indication of stress is determined beat-to-beat and determined as detected when one data sample is classified as indicative of stressed over a time duration of only one heart beat and the RR distance between said one heart beat and the previous heart beat.

3. Non-transitory storage media according to any of the previous claims, wherein the indication of stress is determined as detected when 1-50, 1-20, 1-10, 1-5, 1-2, or only 1 data samples, each of an individual heart beat, are classified as indicative of stressed.

4. Non-transitory storage media according to any of the previous claims, wherein the indication of stress is determined as detected when a majority of data samples, each of an individual heart beat, are classified as indicative of stressed over a predetermined duration of the acquired data.

5. Non-transitory storage media according to any of the previous claims, wherein the indication of stress is determined as detected when a predetermined number of consecutive data samples of individual heart beats are classified as indicative of stressed, in particular 50, 20, 10, 5 or 2 consecutive data samples of individual heart beats are classified as indicative of stressed.

6. Device for on-line detecting stress using individual heart beat ECG features of data acquired from an individual subject, comprising an electronic data processor and data storage media configured for:

> obtaining a data sample of each heart beat individually from the acquired data;
> calculating a set of fiducial heart beat features from each said data sample of an individual heart beat;
> classifying each heart beat data sample as stressed or not-stressed, using a pretrained classifier which was previously trained using the same fiducial features from previously acquired reference data samples each from individual heart beats of said individual subject;
> determining stress as detected when at least one data sample is classified as stressed, wherein the fiducial heart beat features comprise RT, ST, QR, STc, RR, QRS, QTc, STinterval and QTinterval,
> wherein the pretrained classifier is a Linear Support Vector Machine (SVM), a Kernel Support Vector Machine (K-SVM), a K-Nearest Neighbor (K-NN), or a Random Forest classifier.

7. Device according to the previous claim, wherein stress is determined beat-to-beat and determined as detected when one data sample is classified as stressed over a time duration of only one heart beat and the RR distance between said one heart beat and the previous heart beat.

8. Device according to any of the claims 6-7, wherein the device is a wearable device.

**Patentansprüche**

1. Nichtflüchtiges Speichermedium, das Computerprogrammanweisungen enthält, die, wenn sie von einem Prozessor ausgeführt werden, den Prozessor dazu veranlassen, ein Verfahren zur Online-Bestimmung eines Indikators für die Spannung unter Verwendung der Merkmale eines einzelnen Herzschlag-EKG aus von einem Individuum gesammelten Daten auszuführen, umfassend:

> Abrufen einer Datenprobe jedes einzelnen Herzschlags aus den erfassten Daten;
> Berechnen einer Reihe von Herzschlags-Referenzmerkmalen aus jeder der genannten Datenproben eines einzelnen Herzschlags;
> Klassifizieren jeder Datenprobe als Anzeichen für "mit Spannung" oder "ohne Spannung" unter Verwendung eines vortrainierten Klassifikators, der mit den gleichen Herzschlags-Referenzmerkmalen aus vorher aufgezeichneten Referenzdatenproben einzelner Herzschläge des genannten Individuums vortrainiert wurde, und Bestimmen des erkannten Spannungsindikators, wenn mindestens eine Datenprobe eines individuellen Herzschlags als Spannungsindikator klassifiziert wird,
> wobei die Referenzmerkmale des Herzschlags RT, ST, QR, STc, RR, QRS, QTc, ST-Intervall und QT-Intervall umfassen, und
> wobei der vortrainierte Klassifikator eine lineare *Support Vector Machine* (SVM), eine *Kernel Support Vector Machine* (K-SVM), ein *K-Nearest Neighbor* (K-NN) oder ein Random-Forest-Klassifikator ist.

2. Nichtflüchtiges Speichermedium nach dem vorangehenden Anspruch, wobei das Anzeichen auf Spannung von Schlag zu Schlag bestimmt und als erkannt bestimmt wird, wenn eine Datenprobe über einen Zeitraum von nur einem Herzschlag und der RR-Abstand zwischen dem genannten einen Herzschlag und dem vorangehenden Herzschlag als Anzeichen für "mit Spannung" klassifiziert wird.

**3.** Nichtflüchtiges Speichermedium nach einem der vorangehenden Ansprüche, wobei das Anzeichen für Spannung als erkannt gilt, wenn 1-50, 1-20, 1-10, 1-5, 1-2 oder nur 1 Datenprobe, jeweils von einem einzelnen Herzschlag, als Anzeichen für Spannung klassifiziert wird.

**4.** Nichtflüchtiges Speichermedium nach einem der vorangehenden Ansprüche, wobei das Anzeichen für Spannung als erkannt bestimmt wird, wenn eine Mehrheit von Datenproben, jeweils von einem einzelnen Herzschlag, als Anzeichen für Spannung über eine vorbestimmte Dauer der erfassten Daten klassifiziert wird.

**5.** Nichtflüchtiges Speichermedium nach einem der vorangehenden Ansprüche, wobei das Anzeichen für Spannung als erkannt bestimmt wird, wenn eine vorbestimmte Anzahl von aufeinanderfolgenden Datenproben einzelner Herzschläge als Anzeichen für Spannung klassifiziert wird, insbesondere 50, 20, 10, 5 oder 2 aufeinanderfolgende Datenproben einzelner Herzschläge als Anzeichen für Spannung klassifiziert werden.

**6.** Vorrichtung zur Online-Erkennung von Spannung unter Verwendung der Merkmale eines einzelnen Herzschlag-EKG von Daten, die von einem Individuum erfasst wurden, mit einem elektronischen Datenprozessor und Datenspeichermedium, die dafür konfiguriert sind, um:

eine Datenprobe jedes einzelnen Herzschlags aus den erfassten Daten abzurufen;
einer Reihe von Herzschlags-Referenzmerkmalen aus jeder der genannten Datenproben eines einzelnen Herzschlags zu berechnen;
jedes Herzschlags-Datenprobe als Anzeichen für "mit Spannung" oder "ohne Spannung " unter Verwendung eines vortrainierten Klassifikators zu klassifizieren, der mit den gleichen Referenzmerkmalen aus vorher aufgezeichneten Referenzdatenproben einzelner Herzschläge des genannten Individuums vortrainiert wurde;
Spannung als erkannt zu bestimmen, wenn mindestens eine Datenprobe als "mit Spannung" klassifiziert wird, wobei die Referenzmerkmale des Herzschlags RT, ST, QR, STc, RR, QRS, QTc, ST-Intervall und QT-Intervall umfassen,
wobei der vortrainierte Klassifikator eine lineare *Support Vector Machine* (SVM), eine *Kernel Support Vector Machine* (K-SVM), ein *K-Nearest Neighbor* (K-NN) oder ein Random-Forest-Klassifikator ist.

**7.** Vorrichtung nach dem vorangehenden Anspruch, wobei das Anzeichen für Spannung von Schlag zu Schlag bestimmt und als erkannt bestimmt wird, wenn eine Datenprobe über einen Zeitraum von nur einem Herzschlag und der RR-Abstand zwischen dem genannten einen Herzschlag und dem vorangehenden Herzschlag als "mit Spannung" klassifiziert wird.

**8.** Vorrichtung nach einem der Ansprüche 6-7, wobei die Vorrichtung eine tragbare Vorrichtung ist.

**Revendications**

**1.** Moyen de stockage non transitoire incluant des instructions de logiciel informatique qui, lorsqu'elles sont exécutées par un processeur, font en sorte que le processeur exécute un procédé pour la détermination en ligne d'un indicateur indicatif de stress à l'aide de caractéristiques d'ECG de battement cardiaque individuel de données acquises à partir d'un individu, comprenant :

obtenir un échantillon de données de chaque battement cardiaque individuellement à partir des données acquises ;
calculer un ensemble de caractéristiques de battement cardiaque de repère à partir de chacun desdits échantillons de données d'un battement cardiaque individuel ;
classer chaque échantillon de données comme indicateur de stressé ou non stressé, à l'aide d'un classificateur pré-entraîné qui a préalablement été entraîné à l'aide des mêmes caractéristiques de battement cardiaque de repère à partir d'échantillons de données de référence préalablement acquis chacun à partir de battements cardiaques individuels dudit individu ; et
déterminer l'indicateur de stress comme ayant été détecté lorsqu'au moins un échantillon de données d'un battement cardiaque individuel est classé comme indicateur de stressé, dans lequel les caractéristiques de battement cardiaque de repère comprennent RT, ST, QR, Stc, RR, QRS, QTc, IntervalleST et IntervalleQT, et dans lequel le classificateur pré-entraîné est une Machine à Vecteurs de Support linéaire (SVM), une Machine à Vecteurs de Support à Noyau (K-SVM), un Voisin le Plus Proche de K (K-NN), ou un classificateur de Forêt Aléatoire.

**2.** Moyen de stockage non transitoire selon la revendication précédente, dans lequel l'indication de stress est déterminée battement à battement et déterminée comme ayant été détectée lorsqu'un échantillon de données est classé comme indicateur de stressé sur une durée d'un seul battement cardiaque et la distance RR entre ledit seul battement cardiaque et le battement cardiaque précédent.

**3.** Moyen de stockage non transitoire selon l'une quelconque des revendications précédentes, dans lequel l'indication de stress est déterminée comme ayant été détectée lorsque 1-50, 1-20, 1-10, 1-5, 1-2, ou seulement 1 échantillon(s) de données, chacun d'un battement cardiaque individuel, sont classés comme indicateurs de stressé.

**4.** Moyen de stockage non transitoire selon l'une quelconque des revendications précédentes, dans lequel l'indication de stress est déterminée comme ayant été détectée lorsqu'une majorité d'échantillons de données, chacun d'un battement cardiaque individuel, sont classés comme indicateurs de stressé sur une durée prédéterminée des données acquises.

**5.** Moyen de stockage non transitoire selon l'une quelconque des revendications précédentes, dans lequel l'indication de stress est déterminée comme ayant été détectée lorsqu'un nombre prédéterminé d'échantillons de données consécutifs de battements cardiaques individuels sont classés comme indicateurs de stressé, en particulier 50, 20, 10, 5 ou 2 échantillons de données consécutifs de battements cardiaques individuels sont classés comme indicateurs de stressé.

**6.** Dispositif de détection en ligne de stress à l'aide de caractéristiques d'ECG de battement cardiaque individuel de données acquises à partir d'un individu, comprenant un traiteur de données électronique et un moyen de stockage de données configurés pour:

obtenir un échantillon de données de chaque battement cardiaque individuellement à partir des données acquises ;
calculer un ensemble de caractéristiques de battement cardiaque de repère à partir de chacun desdits échantillons de données d'un battement cardiaque individuel ;
classer chaque échantillon de données de battement cardiaque comme stressé ou non stressé, à l'aide d'un classificateur pré-entraîné qui a été préalablement entraîné à l'aide des mêmes caractéristiques de repère des échantillons de données de référence préalablement acquis chacun à partir de battements cardiaques individuels dudit individu ;
déterminer le stress comme ayant été détecté lorsqu'au moins un échantillon de données est classé comme stressé, dans lequel le battement cardiaque de repère comprend RT, ST, QR, Stc, RR, QRS, QTc, IntervalleST et IntervalleQT,
dans lequel le classificateur pré-entraîné est une Machine à Vecteurs de Support linéaire (SVM), une Machine à Vecteurs de Support à Noyau (K-SVM), un Voisin le Plus Proche de K (K-NN), ou un classificateur de Forêt Aléatoire.

**7.** Dispositif selon la revendication précédente, dans lequel le stress est déterminé battement à battement et déterminé comme ayant été détecté lorsqu'un échantillon de données est classé comme stressé sur une durée d'un seul battement cardiaque et la distance RR entre ledit seul battement cardiaque et le battement cardiaque précédent.

**8.** Dispositif selon l'une quelconque des revendications 6-7, dans lequel le dispositif est un dispositif portable.

Fig. 1

Fig. 2

**Fig. 3**

**Fig. 4**

Fig. 5

## REFERENCES CITED IN THE DESCRIPTION

**Non-patent literature cited in the description**

- Changes in ST, QT and RR ECG intervals during acute stress in firefighters: A pilot study. **PAIVA JOANA S et al.** 2016), 38TH ANNUAL INTERNATIONAL CONFERENCE OF THE IEEE ENGINEERING IN MEDICINE AND BIOLOGY SOCIETY (EMBC). IEEE, 16 August 2016, 3378-3381
- **J. CUNHA.** PHealth and wearable technologies: A permanent challenge. *Stud. Health Technol. Inform,* 2012, vol. 177, 185-195
- Changes in ST, QT and RR ECG intervals during acute stress in firefighters: A pilot study. **J. PAIVA ; S. RODRIGUES ; J. CUNHA.** Engineering in Medicine and Biology Society (EMBC), 2016 IEEE 38th Annual International Conference of the. IEEE, 2016, 3378-3381
- **A. VOSS ; R. SCHROEDER ; A. HEITMANN ; A. PETERS ; S. PERZ.** Shortterm heart rate variability - influence of gender and age in healthy subjects. *PloS one,* 2015, vol. 10 (3), e0118308
- **M. BIRKETT.** The Trier Social Stress Test protocol for inducing psychological stress. *Journal of visualized experiments: JoVE,* 2011, vol. 19 (56
- Vital-jacket: A wearable wireless vital signs monitor for patients' mobility in cardiology and sports. **J. CUNHA ; B. CUNHA ; A. PEREIRA et al.** in Pervasive Computing Technologies for Healthcare, 2010 4th International Conference on. IEEE, 2010, 1-2
- Predicting lapses in attention: a study of brain oscillations, neural synchrony and eye measures. **J. PAIVA.** MSc Thesis. University of Coimbra, 2014, 33-36
- **J. CUNHA ; J. PAIVA.** *Biometric Method and Device for Identifying a Person Through an Electrocardiogram (ECG) Waveform - ref. PT109357,* 2016, PT109357
- **J. PAN ; W. TOMPKINS.** A real-time QRS detection algorithm. *Biomedical Engineering, IEEE Transactions on,* 1985, vol. 32 (3), 230-236
- **D. CLIFFORD.** ECG statistics, noise, artifacts, and missing data. *Advanced Methods and Tools for ECG Data Analysis,* 2006, vol. 6, 55-99
- **H. BAZETT.** An analysis of the time-relations of electrocardiograms. *Heart,* 1920, vol. 7, 353-370
- **KOROS A., D.R.P.S ; PANJWANI G. ; INGURGIO V ; D'ARCY J.F.** Complexity in air traffic control towers: A field study part 1. *Complexity factors; Virginia,* 2003, 1-125
- **PAIVA JS ; DIAS D ; CUNHA JPS.** Beat-ID: Towards a computationally low-cost single heartbeat biometric identity check system based on electrocardiogram wave morphology. *PLOS ONE,* 2017, vol. 12 (7), e0180942, https://doi.org/10.1371/journal.pone.0180942